# EUROPEAN PATENT APPLICATION

(11) **EP 3 586 817 A1**
(43) Date of publication of application: **01.01.2020**
(21) Application number: 18197738.0
(22) Date of filing: 28.09.2018
(51) Int. Cl.: A61K 8/31, A61K 8/36, A61Q 11/00, A61K 8/92, A61K 8/19, A61K 8/368

(54) **ALCOHOL-FREE PERFUME AND METHOD FOR MANUFACTURING AN ALCOHOL-FREE PERFUME**

(30) Priority: 27.06.2018 PL 42610518
(71) Applicant: Finea sp. z o.o., 01-350 Warszawa (PL); Politechnika Krakowska Im. Tadeusza Ko Ciuszki, 31-155 Kraków (PL)
(72) Inventor: Gut, Katarzyna, 01-350 Warszawa (PL); Woli ska-Kennard, Katarzyna, Rochester, ME1 3 XS (GB); Sikora, El bieta, 31-988 Kraków (PL); Miastowska, Ma gorzata, 30-438 Kraków (PL); Laso , Elwira, 33-332 Kraków (PL)
(74) Representative: Bartula-Toch, Marta

(57) **Abstract**

The solution according to the invention is that the emulsification process is carried out at a constant temperature in the range of from 20 to 30°C by adding a portion of the aqueous phase in an amount of 30 to 50% by weight composed of a mixture of water and preservative to the emulsifier and fragrance composition mixture. The mixture is vigorously shaken continuously to obtain a homogeneous system. It is then mixed with the speed of the mechanical stirrer in the range of 450 to 550 rpm by dropping the remaining portion of the aqueous phase until a transparent system is obtained. The oil phase is obtained by combining from 5 to 10% by weight of an emulsifier and from 5 to 15% by weight of a fragrance composition, the weight ratio S:O of the emulsifier (S) to the fragrance composition (O) ranging from 1: 1 to 1 : 3. On the other hand, the aqueous phase is obtained by
by combining the preservative in an amount of 0.00025 to 1% by weight with water in an amount to make up to 100% by weight of the ingredients.

## Description

The subject of the invention is a composition of non-alcoholic perfumes with nanoemulsion properties and a method of producing non-alcoholic perfumes.

In the composition of classic perfumery products on the market, ethanol is used as a solvent for hydrophobic fragrances. Introducing lipophilic systems into water without solubilizer (e.g. ethanol) is extremely difficult. Obtaining stable oil-in-water nanoemulsions containing fragrance compositions, without the addition of ethanol or other solvents (co-surfactants) from the group of polyhydric alcohols such as glycols or glycerin is a technological challenge.

Alcohol-based perfumes present on the market are a standard solution, however, ethyl alcohol is a solvent with a specific irritating potential. Perfumery products in the form of solid and perfumed oils, have a number of disadvantages from the utilitarian point of view, namely, it is not possible to disperse the product on the clothes, in the air, they leave greasy and slippery spots on the perfumed surfaces. Discussed perfumery products have their advantages and disadvantages and therefore new solutions are sought for the physico-chemical form of perfumery preparations. Additionally, to expand the market with new customer groups: adolescents, allergy sufferers, Middle East markets, elimination of the most popular solvent of fragrance compositions, i.e. ethyl alcohol, becomes not only a necessity but also a challenge.

Nanoemulsions are a modern form of cosmetic preparations that is an achievement of nanotechnology. From a physico-chemical point of view, these are liquid colloidal systems characterized by a high degree of dispersion in the range of 20 to 500 nm, consisting of an aqueous phase, an oil phase and a surfactant, sometimes with the addition of a co-surfactant. These systems are characterized
by high kinetic stability. Small size of the nanoemulsion particles reduces the effect of the force of gravity, which is overcome by Brownian motion, which prevents the process of sedimentation and creaming. However, from a thermodynamic point of view, nanoemulsions are non-equilibrium systems, which can lead to their destabilization, i.e. flocculation, coalescence and/or Ostwald ripening. In contrast to microemulsions, obtaining them requires the use of smaller amounts of surfactant, which allows to obtain systems that are more safe for the body. Clear appearance, smoothness and low viscosity make nanoemulsions an attractive form for many industrial applications, including cosmetics technology.

In the nanoemulsions described in the literature and obtained on a large scale a single lipophilic component (insoluble in water) is most often used as the oil phase. It should be emphasized that fragrance compositions contain from several to several dozens of compounds with different chemical structures (alcohols, phenols, aldehydes, esters, saturated, unsaturated, cyclic, branched hydrocarbons), making them a difficult base for obtaining stable nanoemulsion systems. Obtaining a water-based perfumes is an extremely difficult task, due to the hydrophobic nature of the fragrance composition and its complex composition.

The main technological problem is to obtain a stable product constituting a mixture of insoluble liquids: water and a hydrophobe fragrance composition in an amount of 5 to 15%, corresponding to the content of the fragrance composition in conventional perfumery products. The nanoemulsion not only acts as a carrier for the fragrance composition but also increases the chemical stability of the compounds in the composition.

The literature describes examples of perfumery products developed on the basis of a safe solvent, i.e. water, so-called "alcohol free". The fragrance compositions used as an essence are by nature a mixture of hydrophobic (water-insoluble) compounds, therefore obtaining stable formulations is very difficult. One of the solutions facilitating the introduction of fragrance compositions into the water is the use of solubilizers, i.e. substances increasing solubility, aroma oils, for example glycols or glycerine, or the use of surface-active compounds of so-called surfactants so as to obtain stable products containing hydrophobic fragrances in the form of emulsions, microemulsions, micelles or liposomes.

Patent literature describes solutions for non-alcoholic perfumes.

Patent application CN103637942 (A) "Alcohol-free transparent perfume composition" discloses a composition with a high concentration of surfactants of more than 10% and the presence of polyhydric alcohols, such as 1,3-butanediol and glycerine.

Patent application US5468725 (A) "Alcohol free perfume" discloses perfumes in the form of a microemulsion containing a high concentration of surfactants, including cationic ones, which irritate the skin.

Patent application US5736505 (A) "Non-alcoholic perfume or cologne" discloses a perfume composition containing an additional solubilizer - glycereth-7-triacetate, at a concentration of from 3 to 12%.

While, EP2127632 A1 "Perfume composition with reduced alcohol content" discloses the composition of glycerine-containing perfumes, an additional hydrophobic solvent, for example, isohexadecane and isoeicosan, and anionic emulsifiers, for example Disodium Lauryl Sulfosuccinate accompanying non-ionic emulsifiers.

The perfume formulations described in the above patents include, in addition to additional solubilizers, such as polyhydroxyl alcohols or paraffinic hydrocarbons, cationic or anionic surfactants that have the potential to irritate the skin.

The essence of the solution according to the invention in the field of non-alcoholic perfumes is that they have the form of oil-in-water nanoemulsion, and include a fragrance composition in an amount of 5 to 15% by weight, the emulsifier from the group non-ionic surfactants in an amount of 5 to 10% by weight, a preservative in an amount of 0.00025 to 1% by weight and water in addition to 100% by weight. The weight ratio S:O of the emulsifier (S) to the fragrance composition (O) ranging from 1:1 to 1: 3.

Preferably, the fragrance composition is a mixture of a dozen or several dozen low molecular weight, volatile, lipophilic substances with a cyclic, aliphatic or aromatic structure.

Preferably the emulsifier is a compound from castor oil oxyethylene derivatives.

Preferably, the emulsifier is a polyglycerol derivative in the form of polyglycerol-4 esters and sebacic and lauryl acids.

Preferably, the emulsifier is a polyglycerol derivative in the form of polyglycerol-6 esters and capric and caprylic acids.

Preferably, a preservative is a microbiological purity stabilizer from the group of nAg silver colloids.

Preferably, a preservative is a microbiological purity stabilizer from the group of nAu gold colloids.

Preferably, the preservative is an anisic acid or salt thereof.

Preferably, the preservative is an a levulinic acid or salt thereof.

The essence of the solution according to the invention in the scope of the method is that the emulsification process is carried out at a constant temperature in the range of from 20 to 30°C by adding a portion of the aqueous phase in an amount of 30 to 50% by weight composed of a mixture of water and preservative to the emulsifier and fragrance composition mixture. The mixture is vigorously shaken continuously to obtain a homogeneous system. It is then mixed with the speed of the mechanical stirrer in the range of 450 to 550 rpm by dropping the remaining portion of the aqueous phase until a transparent system is obtained. The oil phase is obtained by combining from 5 to 10% by weight of an emulsifier and from 5 to 15% by weight of a fragrance composition, the weight ratio S:O of the emulsifier (S) to the fragrance composition (O) ranging from 1:1 to 1:3. On the other hand, the aqueous phase is obtained by combining the preservative in an amount of 0.00025 to 1% by weight with water in an amount to make up to 100% by weight of the ingredients.

Preferably, the fragrance composition is a mixture of a dozen or several dozen low molecular weight, volatile, lipophilic substances with a cyclic, aliphatic or aromatic structure.

Preferably the emulsifier is a compound from castor oil oxyethylene derivatives.

Preferably, the emulsifier is a polyglycerol derivative in the form of polyglycerol-4 esters and sebacic and lauryl acids.

Preferably, the emulsifier is a polyglycerol derivative in the form of polyglycerol-6 esters and capric and caprylic acids.

Preferably, a preservative is a microbiological purity stabilizer from the group of nAg silver colloids.

Preferably, a preservative is a microbiological purity stabilizer from the group of nAu gold colloids.

Preferably, the preservative is an anisic acid or salt thereof.

Preferably, the preservative is an levucic acid or salt thereof.

An advantage of the solution according to the invention is to obtain nanoemulsion formulations compatible with fragrance compositions, without the addition of ethanol or other solvents (co-surfactants) from the polyhydric alcohol group. It is also worth emphasizing that it is not necessary to use an additional solubilizer, for example in the form of a low polarity oil (e.g. isohexadecane), increasing the stability of the system. As emulsifiers, only non-ionic, skin-friendly emulsifiers are used at concentrations that are safe for the body, not exceeding 10%. It should also be emphasized that the pre-emulsification process is carried out at a temperature of from 20 to 30°C, which significantly reduces the cost of the process, because the temperature of the emulsification process in the case of conventional emulsion systems it is usually about 65-75°C. The manufacturing process of the nanoperfumes ensures chemical stability of the fragrance compositions used.

The development of perfumes in the form of a water-based nanoemulsion, eliminates ethanol or other solvents for water. The elimination of alcohol - flammable, and in higher concentrations, irritant solvent - allows to create a safe product for a wider group of consumers. In addition, it is necessary to emphasize the economic aspect related to the international exchange of goods and the reduction of transport costs.

Low viscosity, in the range of from 60 to 110 mPas, facilitates additional application, while the fluid nature of the system gives the nanoemulsion pleasant usable properties.

The object of the inventions is presented in the embodiments.

### Example 1

The components of the oil phase (fragrance composition No. 1) are weighed in an amount of 6% by weight, surfactant (PEG-35 Castor oil, castor oil oxyethylated with 35 moles of ethylene oxide) in an amount of 10% by weight, silver colloid (nAg) in an amount of 0.00025% by weight and water in addition to 100% by weight. The emulsification process is carried out at a constant temperature of from 25°C by adding a portion of the aqueous phase (a mixture of water and stabilizer of microbiological purity) in an amount of 40% by weight to a mixture of a surfactant with a fragrance composition in a given weight ratio. The mixture is vigorously shaken continuously, using a shaker, to obtain a homogeneous system. It is then mixed with the speed of the mechanical stirrer with a rotational speed of agitators of 500 rpm by dropping the remaining portion of the aqueous phase until a transparent system is obtained. The appropriately selected weight ratio of the surfactant to the oil phase ensures a stable nanoemulsion.

A nanoemulsion is obtained with the following composition in % by weight:
- nAg - 0.00025%,
- fragrance composition no. 1 - 6%
- PEG-35 Castor oil - 10%
- water - up to 100%.

### Example 2

The components of the oil phase: fragrance composition No. 2 are weighed in an amount of 6% by weight, surfactant (PEG-35 Castor oil) in an amount of 6% by weight and aqueous phase, silver colloid in an amount of 0.00025% by weight and water in addition to 100% by weight. As a result of the procedures described in Example 1 a nanoemulsion is obtained with the following composition in % by weight:
- nAg - 0.00025%,
- fragrance composition no. 2 - 6%
- PEG-35 Castor oil - 6%
- water - up to 100%.

### Example 3

The components of the oil phase: fragrance composition 3 are weighed in an amount of 15% by weight, surfactant (PEG-35 Castor oil) in an amount of 10% by weight and aqueous phase: silver colloid nAg in an amount of 0.00025% by weight and water in addition to 100% by weight. As a result of the procedures described in Example 1 a nanoemulsion is obtained with the following composition in % by weight:
- nAg - 0.00025%,
- fragrance composition no. 3 - 15%
- PEG-35 Castor oil - 10%
- water - up to 100%.

### Example 4

The components of the oil phase: fragrance composition 4 are weighed in an amount of 6% by weight, surfactant (polyglycerol-4 esters and sebacic and lauryl acids as well as polyglycerol-6 esters and capric and caprylic acids) in an amount of 10% by weight and the components of the aqueous phase: silver colloid nAg in an amount of 0.00025% by weight and water in addition to 100% by weight. As a result of the procedures described in Example 1 a nanoemulsion is obtained with the following composition in % by weight:
- nAu - 0.00025%,
- fragrance composition (4) - 6%
- polyglycerol-4 esters and sebacic and lauryl acids as well as polyglycerol-6 esters and capric and caprylic acids - 10 %
- water - up to 100%.

### Example 5

The components of the oil phase: fragrance composition No. 5 are weighed in an amount of 6% by weight, surfactant (castor oil oxyethylated with 35 moles of ethylene oxide) in an amount of 10% by weight and the components of the aqueous phase: a mixture of sodium salts of levulinic and anisic acid in an amount of 1% by weight and water in addition to 100% by weight.

The emulsification process is carried out at a constant temperature of from 23°C by adding a portion of the aqueous phase (a mixture of water and stabilizer of microbiological purity) in an amount of 50 % by weight to a mixture of a surfactant with a fragrance composition in a given weight ratio. The mixture is vigorously shaken continuously, using a shaker, to obtain a homogeneous system. It is then mixed with the speed of the mechanical stirrer with a rotational speed of agitators of 450 rpm by dropping the remaining portion of the aqueous phase until a transparent system is obtained.

This way a nanoemulsion is obtained with the following composition in % by weight:
- a mixture of sodium salt of anisic acid and a mixture of sodium salt of levulinic and - 1%
- fragrance composition no. 5 - 6 %
- PEG-35 Castor oil - 10 %
- water - up to 100 %

The nanoemulsions obtained in Examples 1 to 5 were characterized by average internal particle size of from 20 to 100 nm, transparent appearance, low viscosity (60 - 100 mPas, T=25°C, v=50
- 1) low surface tension: (28 - 30 mN/m, T= 25°C) and pH corresponding to the physiological pH of the skin.

## Claims

1. Non-alcoholic perfumes **characterized in that** they have the form of oil-in-water nanoemulsion, and include a fragrance composition in an amount of 5 to 15% by weight, the emulsifier from the group of non-ionic surfactants in an amount of 5 to 10% by weight, a preservative in an amount of 0.00025 to 1% by weight and water in addition to 100% by weight, the weight ratio S : O of the emulsifier (S) to the fragrance composition (O) ranging from 1:1 to 1: 3.

2. Perfumes according to claim 1, **characterized in that** the fragrance composition is a mixture of a dozen or several dozen low molecular weight, volatile, lipophilic substances with a cyclic, aliphatic or aromatic structure.

3. Perfumes according to claim 1, **characterized in that** the emulsifier is a compound from castor oil oxyethylene derivatives.

4. Perfumes according to claim 1, **characterized in that** the emulsifier is a polyglycerol derivative in the form of polyglycerol-4 esters and sebacic and lauryl acids.

5. Perfumes according to claim 1, **characterized in that** the emulsifier is a polyglycerol derivative in the form of polyglycerol-6 esters and capric and caprylic acids.

6. Perfumes according to claim 1, **characterized in that** a preservative is a microbiological purity stabilizer from the group of nAg silver colloids.

7. Perfumes according to claim 1, **characterized in that** a preservative is a microbiological purity stabilizer from the group of nAu gold colloids.

8. Perfumes according to claim 1, **characterized in that** a preservative is an anisic acid or salt thereof.

9. Perfumes according to claim 1, **characterized in that** a preservative is an levulinic acid or salt thereof.

10. A method of producing non-alcoholic perfumes **characterized in that** the emulsification process is carried out at a constant temperature in the range of from 20 to 30°C by adding a portion of the aqueous phase in an amount of 30 to 50% by weight composed of a mixture of water and preservative to the emulsifier and fragrance composition mixture, then the mixture is vigorously shaken continuously to obtain a homogeneous system, it is then mixed with the speed of the mechanical stirrer in the range of 450 to 550 rpm by dropping the remaining portion of the aqueous phase until a transparent system is obtained, wherein the oil phase is obtained by combining from 5 to 10% by weight of an emulsifier and from 5 to 15% by weight of a fragrance composition, the weight ratio S: O of the emulsifier (S) to the fragrance composition (O) ranging from 1: 1 to 1 : 3, on the other hand, the aqueous phase is obtained by combining the preservative in an amount of 0.00025 to 1% by weight with water in an amount to make up to 100% by weight of the ingredients.

11. A method according to claim 11, **characterized in that** the fragrance composition is a mixture of a dozen or several dozen low molecular weight, volatile, lipophilic substances with a cyclic, aliphatic or aromatic structure.

12. A method according to claim 11, **characterized in that** the emulsifier is a compound from castor oil oxyethylene derivatives.

13. A method according to claim 11, **characterized in that** the emulsifier is a polyglycerol derivative in the form of polyglycerol-4 esters and sebacic and lauryl acids.

14. A method according to claim 11, **characterized in that** the emulsifier is a polyglycerol derivative in the form of polyglycerol-6 esters and capric and caprylic acids.

15. A method according to claim 11, **characterized in that** a preservative is a microbiological purity stabilizer from the group of nAg silver colloids.

16. A method according to claim 11, **characterized in that** a preservative is a microbiological purity stabilizer from the group of nAu gold colloids.

17. A method according to claim 11, **characterized in that** a preservative is an anisic acid or salt thereof.

18. A method according to claim 11, **characterized in that** a preservative is an a levulinic acid or salts thereof.
